# EUROPEAN PATENT SPECIFICATION

(11) **EP 0 678 096 B1**
(45) Date of publication and mention of the grant of the patent: **19.03.1997**
(21) Application number: 94906568.4
(22) Date of filing: 07.01.1994
(51) Int. Cl.: C07H 21/00

(54) **SYNTHESIS OF DIMMER BLOCKS AND THEIR USE IN ASSEMBLING OLIGONUCLEOTIDES**
SYNTHESE VON DIMER-BLÖCKEN UND IHRE VERWENDUNG BEI DER ZUSAMMENSETZUNG VON OLIGONUKLEOTIDEN
SYNTHESE DE BLOCS DIMERES ET LEUR UTILISATION POUR L'ASSEMBLAGE D'OLIGONUCLEOTIDES

(30) Priority: 08.01.1993 US 2823
(43) Date of publication of application: 25.10.1995
(73) Proprietor: HYBRIDON, Inc., Worcester, Massachusetts 01605 (US)
(72) Inventor: TANG, Jin-Yan, Shrewsbury, MA 01545 (US); IADAROLA, Patricia L., Worcester, MA 01604 (US); AGRAWAL, Sudhir, Shrewsbury, MA 01545 (US)
(74) Representative: Vossius, Volker, Dr.
(86) International application number: US9400296
(87) International publication number: WO9415946

(56) References cited:
- EP-A- 0 136 543
- EP-A- 0 219 342
- EP-A- 0 241 363
- EP-A- 0 386 987
- EP-A- 0 552 766
- WO-A-91/06556
- WO-A-91/16902
- WO-A-92/04358
- WO-A-92/20967
- WO-A-94/02499
- US-A- 4 469 863
- US-A- 5 003 097
- TETRAHEDRON LETTERS. vol. 32, no. 37 , 9 September 1991 , OXFORD GB pages 4981 - 4984 J.HELINSKI ET AL. 'N,N-Diisopropyl-O-p-nit rophenyl-P-methylphosphonoamidate: Novel Difunctional Piii Reagent in Oligonucleoside Methylphosphonate Synthesis Containing 4-Nitrophenoxy Group.'
- TETRAHEDRON LETTERS. vol. 33, no. 17 , 21 April 1992 , OXFORD GB pages 2357 - 2360 H.C.P.F.ROELEN ET AL. 'Synthesis of Alkylphosphon(othio)ate Analogues of DNA.'
- Nucleic Acids Research 16, 7633-45, 1988
- Journal of Organic Chemistry 55, 4693-99, 1990
- Advanced Organic Chemistry by J. March (1985) pages 16-18, 237-9
- Declaration of Dr. Sudhir Agrawal

## Description

The invention relates to the chemical synthesis of oligonucleotides. More particularly, the invention relates to the synthesis of oligonucleotides having modified internucleotide linkages.

Since the discovery by Zamecnik and Stephenson, Proc. Natl. Acad. Sci. USA 75: 280-284 (1978), that synthetic oligonucleotides can inhibit Rous sarcoma virus replication, there has been great interest in the use of oligonucleotides and oligonucleotide analogs having modified internucleotide linkages to control gene regulation and to treat pathological conditions.

Various methods have been developed for the synthesis of oligonucleotides for such purposes. Early synthetic approaches included phosphodiester and phosphotriester chemistries. Khorana et al., J. Molec. Biol. 72: 209 (1972) discloses phosphodiester chemistry for oligonucleotide synthesis. Reese, Tetrahedron Lett. 34: 3143-3179 (1978) discloses phosphotriester chemistry for synthesis of oligonucleotides and polynucleotides. These early approaches have largely given way to the more efficient phosphoramidite and H-phosphonate approaches to synthesis. Beaucage and Caruthers, Tetrahedron Lett. 22: 1859-1862 (1981) discloses the use of deoxynucleoside phosphoramidites in polynucleotide synthesis. Agrawal and Zamecnik, U.S. Patent No. 5,149,798 (1992) discloses optimized synthesis of oligonucleotides by the H-phosphonate approach.

Both of these modern approaches have been used to synthesize oligonucleotides having a variety of modified internucleotide linkages. Agrawal and Goodchild, Tetrahedron Lett. 28: 3539-3542 (1987) teaches synthesis of oligonucleotide methylphosphonates using phosphoramidite chemistry. Connolly et al., Biochemistry 23: 3443 (1984) discloses synthesis of oligonucleotide phosphorothioates using phosphoramidite chemistry. Jager et al., Biochemistry 27: 7237 (1988) discloses synthesis of oligonucleotide phosphoramidates using phoshoramidite chemistry. Agrawal et al., Proc. Natl. Acad. Sci. USA 85: 7079-7083 (1988) discloses synthesis of oligonucleotide phosphoramidates and phosphorothioates using H-phosphonate chemistry.

Both phosphoramidite and H-phosphonate chemical syntheses are carried out on a solid support that is stored in a reaction vessel. The required reaction steps for coupling each nucleotide are detritylation, coupling, capping, and oxidation. For small scale (up to 1 µmmole) synthesis, the total time for the addition of one nucleotide is about 6 minutes. An oligonucleotide, 30-mer in length, can be assembled in 180 minutes. Under these conditions, synthesized oligonucleotides are chemically pure and biologically active. However, when oligonucleotides are synthesized on a larger scale (up to 1 mmole), the time for addition of each nucleotide onto CPG is in the range of 50 to 60 minutes, requiring approximately 25 hours for assembling a 25-mer oligonucleotide. The increase in time is because of the volume of the solid support being used in synthesis. This increase in cycle time exposes the already assembled oligonucleotide sequence to all reaction steps (including dichloroacetic acid step, coupling step, oxidation step and capping step) for a longer time. This increase in total assembly time affects the yield as well as chemical and biological properties of the compound. The chemical and biological properties are mainly affected by depurination, base modifications, and the like.

To reduce the effects of these problems, it is possible to synthesize oligonucleotides using dimeric or multimeric synthons, thereby reducing the number of cycles, and thus the time required for synthesizing oligonucleotides. To this end, several investigators have worked toward developing acceptable dimeric or multimeric synthon approaches. Khorana, Science 203: 614-625 (1979) introduced the concept of multimeric synthons, using a phosphodiester approach. Crea and Itakura, Proc. Natl. Acad. Sci. USA 75: 5765-5769 (1978); Reese, Tetrahedron Lett. 34: 3143-3179 (1978); and Ohtsuka et al., Nucleic Acids Res. 10: 6553-6570 (1982) all disclose use of dimeric or multimeric synthons in a phosphotriester approach. Kumar and Poonian, J. Org. Chem. 49: 4905-4912 (1984); and Wolter et al., Nucleosides and Nucleotides 5: 65-77 (1986) disclose phosphoramidite synthesis of oligonucleotide phosphodiesters using dimeric synthons. Marugg et al., Nucleic Acids Res. 12: 9095-9110 (1984) teaches use of a dinucleotide thiophosphotriester to produce oligonucleotides containing one phosphorothioate linkage. Connolly et al., Biochemistry 23: 3443 (1984); and Cosstick and Eckstein, Biochemistry 24: 3630 (1985) disclose addition of one dinucleotide phosphorothioate to a growing oligonucleotide chain using a phosphoramidite approach. Brill and Caruthers, Tetrahedron Lett. 28: 3205 (1987) discloses synthesis of thymidine dinucleotide methylphosphonothioates. Roelen et al., Nucleic Acids Res. 16: 7633-7645 (1988) discloses use of the dimeric nucleotide methylphosphonothioate TA to introduce a single methylphosphonothioate linkage into a short hexameric oligonucleotide in a solution phase reaction.

WO 92/04358 describes a reagent comprising a solution of sulfur and a tertiary amine, in a suitable solvent such as carbon disulfide suitable for sulfurizing an organophosphite. Furthermore, this document describes a process in which phosphites and phosphonous esters are sulfurized to their corresponding phosphorothioates or phosphonothioates, respectively, using the described sulfurizing agent.

US 5,003,097 and lyer et al. in J. Org. Chem. **55**, 4693-4699 (1990) describe the use of 3H-1,2-benzodithiol-3-one-1,1-dioxide as a sulfurizing agent in the synthesis of nucleotide dimers with a phosphorothioate internucleotide linkage.

The foregoing studies represent progress in the development of methods for synthesizing oligonucleotides using dimeric or multimeric synthons. Unfortunately, certain deficiencies remain extant. For example, the existing chemistries for producing dimeric nucleotides containing phosphorothioate or methyl- phosphonothioate internucleotide linkages are highly complex. Thus, only two methylphosphonothioate-containing dimers (TT and TA) have been reported to date. In addition, none of the existing dimeric synthons have been successfully used to synthesize oligonucleotides having exclusively alkylphosphonate, phosphoramidate, phosphorothioate, or alkylphosphonothioate internucleotide linkages. There is, therefore, a need for new methods employing simplified chemistries for the synthesis of dimeric synthons, as well as for new methods for using such dimeric synthons to assemble oligonucleotides.

Thus, the technical problem underlying the present invention is to provide an efficient process for preparing dimeric alkylphosphonothioate nucleotides in high yields in a single pot solution phase reaction.

These dimeric nucleotides (dimer blocks) also give rise to a method of using such dimeric nucleotides to assemble oligonucleotides containing alkylphosphonothioate linkages. Moreover, the dimer blocks according to the invention provide a method of using dimer blocks to assemble oligonucleotides having exclusively alkylphosphonate, phosphoramidate, phosphorothioate or alkylphosphonothioate internucleotide linkages, or mixtures thereof.

The process of the present invention provides efficient methods that reduce total assembly time of oligonucleotides. Futhermore, the process of the present invention provides efficient methods that reduce total solvent consumption required for oligonucleotide assembly. Moreover, the process of the present invention provides efficient methods that ease purification of oligonucleotides by increasing the yield of full length oligonucleotides. Additionally the process of the present invention provides efficient methods that reduce side reactions by reducing the exposure of partially assembled oligonucleotides to chemicals. Finally, the process of the present invention reduces overall cost of oligonucleotide assembly by allowing the use of inexpensive solution phase chemistry to achieve half of the total synthesis.

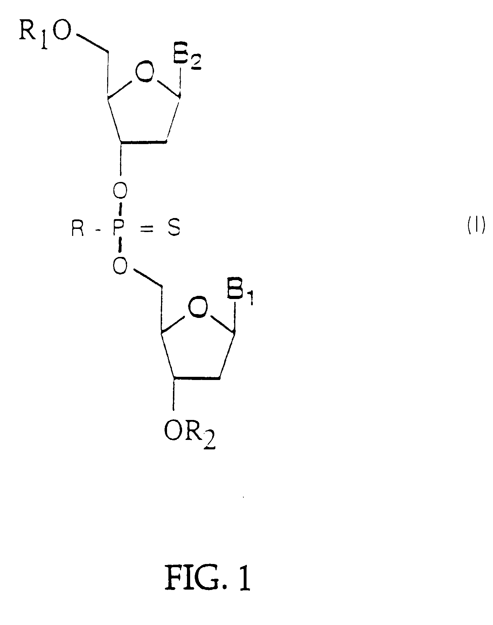

Figure 1 shows a dimeric alkylphosphonothioate nucleotide of the general formula (I) according to the invention, wherein R₁ is a protective group such as dimethoxytrityl, mononmethoxytrityl or trityl, R₂O is a hydroxyl group, or R₂ is a protective group such as levulinyl, t-butyldimethylsilyl, B₁ is G, A, T, or C, and B₂ is G, A, T, or C and R is an alkyl group.

The invention relates to reagents and methods for assembling oligonucleotides. More particularly, the invention relates to the assembly of oligonucleotides having modified internucleotide linkages.

The present invention provides a new process for making dimer blocks. Dimer blocks are dimeric nucleotides having modified internucleotide linkages and blocking groups at the 5'-hydroxyl. Preferred blocking groups include tert-butyldi-methylsilyl, dimethoxytrityl, levulinyl, monomethoxytrityl and trityl groups. The 3' position of the dimer block may have a blocking group, a free hydroxyl, an H-phosphonate, a phosphotriester, or a β-cyanoethylphosphoramidite group. The Preferred modified internucleotide linkage is an alkylphosphonothioate linkage. The modified linkage of the dimer block has an alkyl group.

In a very general sense, the method of synthesizing dimer blocks according to the invention can be considered to be a method of synthesizing a dimer block having an alkylphosphonothioate internucleotide linkage, the method comprising the steps of:
(a) condensing together a first nucleoside derivative having a protective group at a 5' end and a condensing group at a 3' end with a second nucleoside derivative having a protective group at a 3' end and a hydroxyl group at a 5' end to form a dinucleotide derivative having a reduced internucleotide linkage, and
(b) oxidizing the internucleotide linkage with Beaucage reagent to yield a dimer block alkylphosphonothioate.

The precise dimer block obtained, of course will depend upon the nature of the first nucleoside derivative.

In the process the present invention, a dimer block is obtained having 5' and 3' blocking groups and an alkylphosphonothioate internucleotide linkage. In this process, the method comprises the steps of (a) joining together, by phosphoramidite chemistry, a nucleoside having a 5' blocking group and a nucleoside having a 3' blocking group, and (b) adding Beaucage reagent (3H-1,2-benzodithiol-3-one-1,1-dioxide).

In a second embodiment of this aspect of the invention, the method produces a dimer block having a 5' blocking group, a 3' free hydroxyl group, and a phosphorothioate internucleotide linkage. In this embodiment, the method comprises steps (a) and (b) of the first embodiment above, and further comprises the step of (c) deprotecting the 3'-hydroxyl group. This is achieved by the use of conditions selective for removal of the 3' protective group only. For example, if the 5' protective group is dimethoxytrityl, monomethoxytrityl or trityl, and the 3' group is tert-butyldimethylsilyl, then selective removal of the 3' group is obtained by treatment with tetrabutylammonium fluoride. Alternatively, if the 5' group is dimethoxytrityl, monomethoxytrityl or trityl and the 3' group is levulinyl, selective removal of the 3' group is obtained by treatment with hydrazine hydrate in pyridine/acetic acid.

The process of the present invention offers a method of producing dimer block products that are useful as intermediates for assembling oligonucleotides having modified internucleotide linkages. The ability to produce these dimer blocks in a one pot reactions greatly simplifies their production.

The present invention provides dimer block products having a 5' blocking group, a modified internucleotide linkage and a 3' group that may be a blocking group, or a free hydroxyl. The method for producing these dimer blocks is independent of the sequence of the nucleotides in the dimer block, thus allowing production of all possible dimer sequences containing an alkylphosphonothioate linkage, i.e., GG, GA, GT, GC, AG, AA, AT, AC, TG, TA, TT, TC, CG, CA, CT, and CC. Such dimers are illustrated in Figure 1.

Dimer blocks having modified internucleotide linkages (i. e., phosphorothioate, alkylphosphonate, phosphoramidate, or alkylphosphonothioate), are used to assemble oligonucleotides having such modified internucleotide linkages (dimer blocks having 5' blocking groups and 3' β-cyanoethyl phosphoramidite or H-phosphonate groups are used). Synthesis is then carried out according to either the phosphoramidite or H-phosphonate approach. Support of oligonucleotide synthesis with dimer blocks can be soluble polymers as well as insoluble CPG and polymer beads. Those skilled in the art will recognize that this approach also allows the convenient synthesis of mixed phosphate backbone oligonucleotides, e.g., oligonucleotides having both phosphorothioate and alkylphosphonothioate or phosphodiester and alkylphosphonothioate or phosphodiester and alkylphosphonothioate linkages or any combination of the modified linkages taught herein with each other or with phosphodiester linkages.

The method according to this aspect of the invention provides several advantages over monomeric synthesis of oligonucleotides. First, since half as many assembly cycles are required, the total assembly time is reduced by half, which for large scale synthesis can be a saving of 12 hours or more for a single oligonucleotide. This reduction in time also results in fewer side reactions, since partially assembled oligonucleotides are exposed to chemicals for a shorter time. The method also facilitates purification of oligonucleotides by increasing the proportion of full length oligonucleotides, since that proportion varies inversely with the number of cycles performed. Finally, the method reduces cost of synthesis by cutting solvent consumption by half and by allowing one half of the total synthesis to be carried out using inexpensive solution phase chemistry. The present method extends these advantages to oligonucleotides having exclusively, alkylphosphonothioate linkages as well as to oligonucleotides having any combination of phosphorothioate, alkylphosphonate, phosphoramidate, or alkylphosphonothioate.

The following examples are intended to further illustrate certain preferred embodiments of the methods according to the invention, and are not intended to be limiting in nature.

### Example 1

### Solution Phase Synthesis Of 5'-O-dimethoxytritylthymidyl-3'-O-methyl phosphonothioate-5'-O-thymidine

The synthetic steps for this dimer block for phosphoramidite synthesis of alkylphosphonothioate-containing oligonucleotides are shown in Figure 5.

A mixture of 5'-O-dimethoxytrityl-thymidine-3'-methyl N,N-diisopropyl phosphoramidite (0.5 g, 0.74 mmol) and 3'-O-levulinyl-thymidine (0.25 g, 0.7 mmol) was dissolved in anhydrous acetonitrile (7 ml) and a solution of tetrazole (0.45 M, 3.5 ml) was added. The reaction mixture was stirred for 30 minutes at room temperature, treated with Beaucage reagent (0.29 g in anhydrous acetonitrile, 10 ml) for 15 minutes and then evaporated to remove most of the solvent. The crude reaction product was extracted with dichloromethane and washed with brine. After removal of dichloromethane, the material was redissovled in 7.5 ml pyridine, and 7.5 ml 1 M hydrazine hydrate solution in pyridine/acetic acid (3/2) was added. After 5 minutes, the reaction mixture was cooled in an ice-bath and 2 ml acetyl acetone were added to quench the excess amount of hydrazine hydrate. The mixture was evaporated to a small volume and then directly loaded to a silica gel column (2.5 x 25 cm). The dimer block product, 22, was eluted by using 0 to 5% methanol in dichloromethane (0.5% pyridine) to obtain 0.5g (82% yield). ³¹P NMR = 97.7, 99.0.

## Claims

1. A process for preparing dimeric nucleotides of the general formula (I), 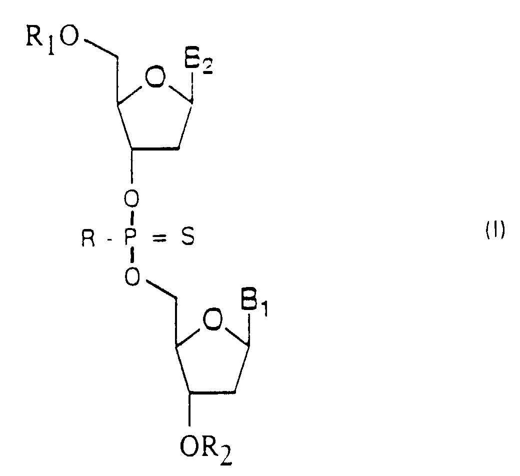 wherein R is an alkyl group, R₁ is a protective group, R₂ is a protective group, B₁ is G, A, T or C, and B₂ is G, A, T or C,
the process comprising the steps of:
a) condensing in solution a 5' protected nucleoside-3'-alkyl-N,N-diisopropyl phosphoramidite of the general formula (II) 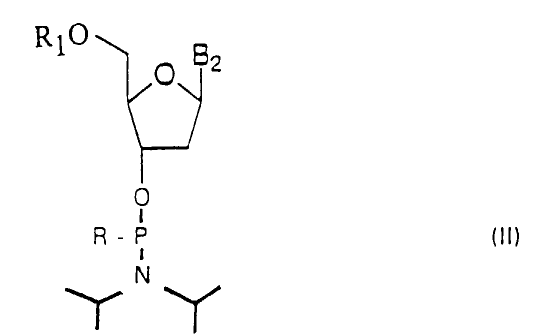 with a nucleoside of the general formula (III), 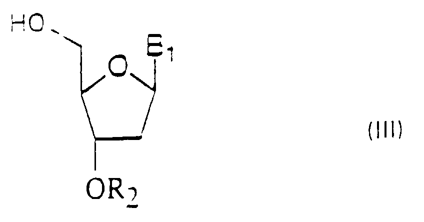 and
b) oxidizing in solution the formed dinucleotide derivative of the general formula (IV) 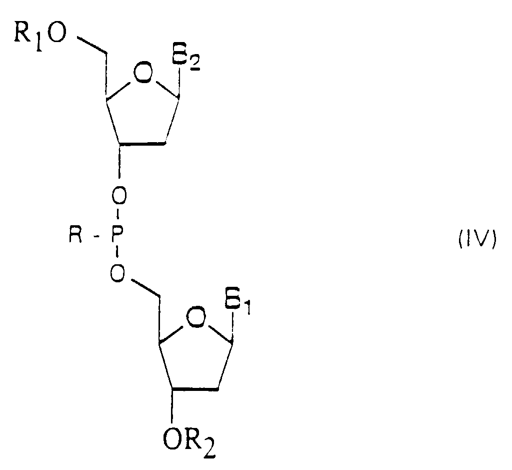 with 3H-1,2-benzodithiol-3-one-1,1-dioxide.

2. The process according to claim 1 for preparing dimeric nucleotides of the general formula (I), wherein R₂ is a hydrogen atom,
the process further comprising the step of:
c) deprotecting the 3'-hydroxyl group.

3. The process according to claim 2, wherein deprotection of the 3'-hydroxyl group is carried out by treatment with hydrazine hydrate in pyridine/ acetic acid,
if the 3' protective group is levulinyl and the 5' protective group is dimethoxytrityl, monomethoxytrityl or trityl.

4. The process according to claim 2, wherein deprotection of the 3'-hydroxyl group is carried out by treatment with tetrabutylammonium fluoride, if the 3' protective group is tert.-butyldimethylsilyl and the 5' protective group is dimethoxytrityl, monomethoxytrityl or trityl.

5. The process according to claim 2 to 4 comprising further the step of purifying the deprotected dimeric nucleotide by silica gel column chromatography and eluting with 0 to 5% methanol in dichloromethane containing 0.5% pyridine.

## Patentansprüche

1. Ein Verfahren zur Herstellung dimerer Nucleotide der allgemeinen Formel (I) 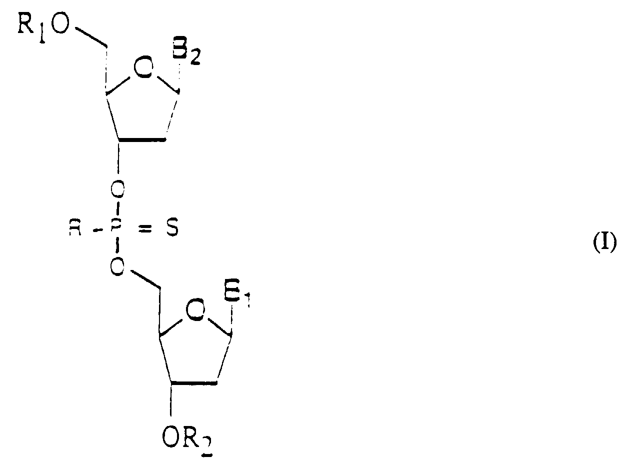 in der der Rest R ein Alkylrest, der Rest R₁ eine Schutzgruppe, der Rest R₂ eine Schutzgruppe und der Rest B₁ G, A, T oder C und der Rest B₂ G, A, T oder C ist, wobei das Verfahren die Schritte umfaßt:
a) Kondensieren in Lösung eines 5'-geschützten Nucleosid-3'-Alkyl-N,N-diisopropylphosphoramidit der allgemeinen Formel (II) 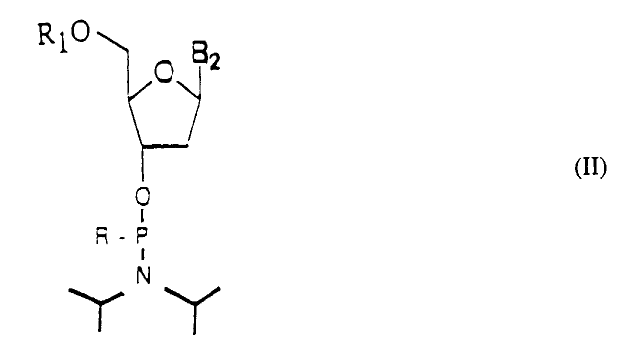 mit einem Nucleosid der allgemeinen Formel (III) 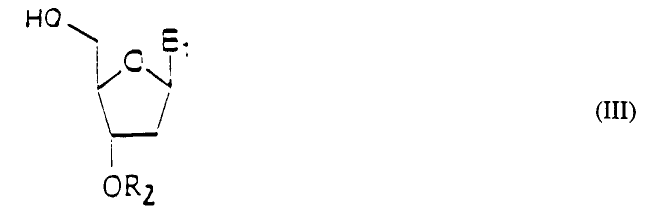 und
b) Oxidieren in Lösung des gebildeten Dinucleotidderivates der allgemeinen Formel (IV) 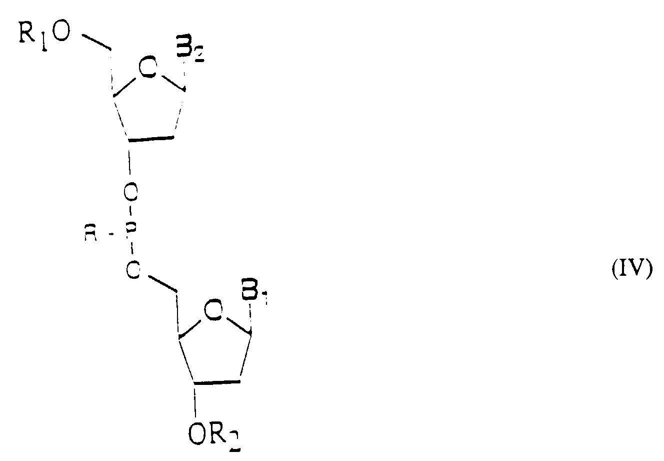 mit 3H-1,2-Benzodithiol-3-on-1,1-dioxid.

2. Verfahren nach Anspruch 1, zur Herstellung dimerer Nucleotide der allgemeinen Formel (I), wobei der Rest R₂ ein Wasserstoffatom ist, das Verfahren weiter den Schritt umfaßt:
c) Entfernen der Schutzgruppe der 3'-Hydroxylgruppe.

3. Verfahren nach Anspruch 2, wobei das Entfernen der Schutzgruppe der 3'-Hydroxylgruppe durch Behandlung mit Hydrazinhydrat in Pyridin/Essigsäure durchgeführt wird, wenn die 3'-Schutzgruppe ein Lävulinylrest und die 5'-Schutzgruppe ein Dimethoxytrityl-, Monomethoxytrityl- oder Tritylrest ist.

4. Verfahren nach Anspruch 2, wobei das Entfernen der Schutzgruppe der 3'-Hydroxylgruppe durch Behandlung mit Tetrabutylammoniumfluorid durchgeführt wird, wenn die 3'-Schutzgruppe ein tert.-Butyldimethylsilylrest ist und die 5'-Schutzgruppe ein Dimethoxytrityl-, Monomethoxytrityl- oder Tritylrest ist.

5. Verfahren nach Anspruch 2 bis 4, weiter umfassend den Schritt der Reinigung des dimeren Nucleotids, von dem die Schutzgruppe entfernt worden ist, durch Silicagel-Säulenchromatographie und Eluieren mit 0 bis 5% Methanol in Dichlormethan, 0,5% Pyridin enthaltend.

## Revendications

1. Procédé pour préparer des nucléotides dimères répondant à la formule générale (I) suivante : 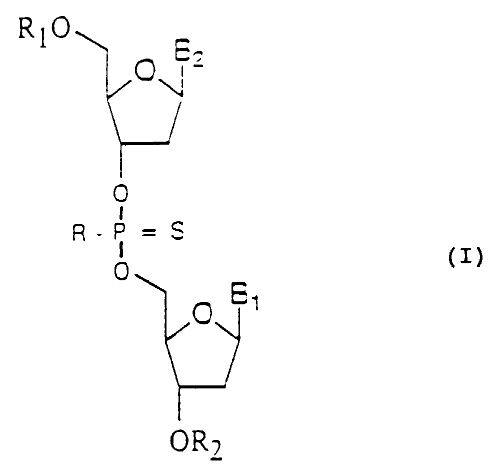 dans laquelle R représente un groupe alkyle, R₁ représente un groupe protecteur, R₂ représente un groupe protecteur, B₁ représente G, A, T ou C et B₂ représente G, A, T ou C, ledit procédé comprenant les étapes consistant à :
a) effectuer une réaction de condensation, en solution, entre un nucléoside-3'-alkyl-N,N-diisopropylphosphoramidite protégé en 5' répondant à la formule générale (II) suivante : 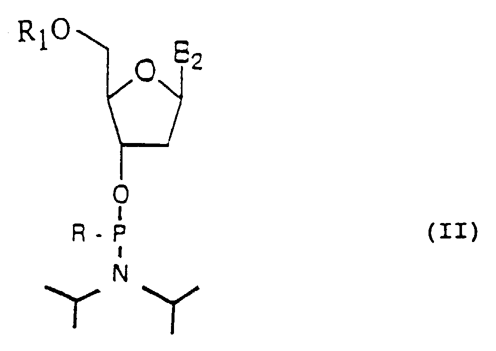 et un nucléoside répondant à la formule générale (III) suivante : 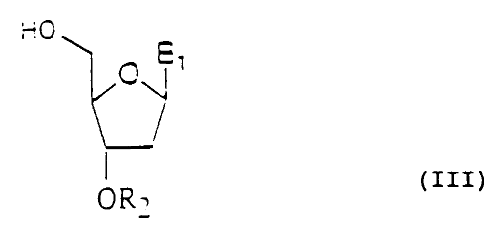
b) oxyder, en solution, le dérivé de dinucléotide obtenu répondant à la formule générale (IV) suivante : 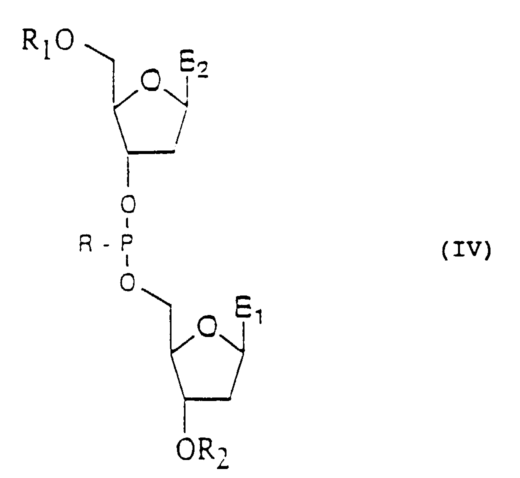 par le 3H-1,2-benzodithiol-3-one-1,1-dioxyde.

2. Procédé selon la revendication 1 pour préparer des nucléotides dimères répondant à la formule générale (I), dans laquelle R₂ représente l'atome d'hydrogène, ledit procédé comprenant en outre l'étape consistant à :
c) déprotéger le groupe hydroxyle en 3'.

3. Procédé selon la revendication 2, selon lequel la déprotection du groupe hydroxyle en 3' est effectuée par un traitement par de l'hydrate d'hydrazine dans un mélange pyridine/acide acétique, quand le groupe protecteur en 3' consiste en le groupe lévulinyle et le groupe protecteur en 5' consiste en l'un des groupes choisis parmi le diméthoxytrityle, monométhoxytrityle et trityle.

4. Procédé selon la revendication 2, selon lequel la déprotection du groupe hydroxyle en 3' est effectuée par un traitement par du flurorure de tétrabutylammonium, quand le groupe protecteur en 3' consiste en le groupe tertiobutyldiméthylsilyle et le groupe protecteur en 5' consiste en l'un des groupes choisis parmi le diméthoxytrityle, monométhoxytrityle et trityle.

5. Procédé selon l'une des revendications 2 à 4, comprenant en outre une étape consistant à purifier le nucléotide dimère déprotégé obtenu, par chromatographie sur colonne de gel de silice par une élution avec de 0 à 5% de méthanol dans du dichlorométhane contenant 0,5% de pyridine.
